# EUROPEAN PATENT SPECIFICATION

(11) **EP 0 368 209 B1**
(45) Date of publication and mention of the grant of the patent: **16.02.1994**
(21) Application number: 89120516.3
(22) Date of filing: 06.11.1989
(51) Int. Cl.: C12M 1/40

(54) **A process for immobilizing an enzyme on an electrode surface**
Verfahren zur Immobilisierung eines Enzyms auf einer Elektrodenoberfläche
Procédé d'immobilisation d'un enzyme sur la surface d'une électrode

(30) Priority: 08.11.1988 JP 282721/88
(43) Date of publication of application: 16.05.1990
(73) Proprietor: NEC CORPORATION, Tokyo (JP)
(72) Inventor: Karube, Isao, Kawasakishi Kanagawa (JP); McNeil, Vincent M., Somerville, MA 02139 (US); Murakami, Toru c/o NEC Corporation, Minato-ku Tokyo (JP)
(74) Representative: Pätzold, Herbert, Dr.-Ing.

(56) References cited:
- EP-A- 0 304 494
- JOURNAL OF THE CHEMICAL SOCIETY, CHEMICAL COMMUNICATIONS, no. 15, 01 August 1988, London (GB); C. IWAKURA et al., pp. 1019-1020#
- CHEMICAL ABSTRACTS, vol. 109, 1988, Columbus, OH (US); Y. IKARIYAMA et al., p. 410, no. 225991t#
- CHEMICAL ABSTRACTS, vol. 108, 1988, Columbus, OH (US); Y. IKARIYAMA et al., 296, no. 18602f#
- CHEMICAL ABSTRACTS, vol. 105, 1986, Columbus,OH (US); M. UMAN et al., no. 183095p#

## Description

The present invention relates to a process for immobilizing an enzyme of an electrode surface and to a biosensor comprising an immobilized enzyme electrode.

A biosensor comprising an electrode on the surface of which an enzyme and a mediator of electron transfer are immobilized was described by Anthony E.G. Cass, et al. in "Analytical Chemistry" Vol. 56 (1984), No. 4, pages 667-671. This biosensor is one that was prepared by depositing, as a mediate of electron transfer, 15 µL of 1,1'-dimethylferrocene (0.1M in toluene) onto the surface of graphite electrodes (4 mm diameter), which had been heated at 100°C in air for 40 hours and allowed to cool; air-drying the deposited electrodes; immersing the dried electrodes into an aqueous solution of 1-cyclohexyl-3-(2-morpholinoethyl) carbodiimide p-methyl-toluenesulfonate for 80 minutes at 20°C, and then into an acetate buffer solution (0.1M, pH 9.5) containing glucose oxidase (12.5 mg/mL) for 90 min at 20°C; and covering the electrodes with a polycarbonate membrane.

A biosensor comprising an electrode on the surface of which an enzyme is immobilized by an electropolymerizing substance was described by Mirtha Umana, et al. in "Analytical Chemistry" Vol. 58 (1986), No. 14, pages 2979-2983. This biosensor is one that was prepared by potentiostatically electrolyzing an aqueous solution of KCl (0.1M), pyrrole (0.5M) and glucose oxidase (500 units/mL) by using platinum or glassy carbon working electrode and Ag/AgCl reference electrode at the potential 0.65 V.

A biosensor comprising an electrode on the surface of which a micro-platinized layer is deposited and then an enzyme is immobilized thereon was described by Yoshihito Ikariyama, et al. in "Analytical Letters" Vol. 20 (1987), No. 7, pages 1407-1416. This biosensor is one that was prepared by platinizing a platinum electrode (5-200 µm diameter) in an aqueous solution of hexachloroplatinate (33 mg/mL) and lead acetate (0.6 mg/mL), by potentiostatic or galvanostatic electrolytic reduction; and then immersing the platinized platinum electrode in a phosphate buffered solution containing glucose oxidase (1.5 mg, 110 unit/mg) for 10 minutes.

EP-A-0 304 494 discloses a glucose-biosensor made from an electroplated electrode impregnated with glucose-oxidase which is chemically cross-linked or else coated with a cross-linked polymer like bovine serum albumin or heparin.

These known biosensors prepared by prior art enzyme immobilization methods however have problems of a short lifetime owing to leaching of the immobilized enzyme or mediator of electron transfer out of the electrode surface, and a narrow response range owing to insufficiency of absolute amounts of them as actually immobilized.

### SUMMARY OF THE INVENTION

An object of the present invention is therefore to present a process for immobilizing an enzyme on an electrode surface that enables to obtain a biosensor having a long lifetime and a wide response range, which biosensor has sufficient amounts of an enzyme and a mediator of electron transfer as actually immobilized on the electrode surface and which biosensor is capable of preventing leaching of them out of the electrode surface.

Said object may be obtained by the process of the independent claims 1 or 2.

Preferred embodiments of the invention according to the features of the independent claims 1 and 2 are mentioned in the subclaims 3 to 5.

Through said stages (a) and (b) of the independent claim 1, obtainable is a biosensor comprising an immobilized enzyme electrode which has, on the electrode surface a layer of an electroplated metal containing the enzmye and a mediator of electron transfer, and a layer of an electropolymerized substance containing the enzyme and the mediator of electron transfer, in this order.

Through said stages (a), (b1), and (b2) of the independent claim 2, obtainable is a bionsensor comprising an immobilized enzyme electrode which has, on the electrode surface, a layer of an electroplated metal containing the enzyme, and a layer of an electropolymerized substance containing a mediator of electron transfer, in this order.

In order to obtain a uniform electroplated metal layer, it is advantageous to subject the electrode surface to an electrochemical pretreatment before the electroplating step (a).

The physical adsorption stage (b₁) may be effectively performed when an electric potential is charged.

In view of solubility, the polymer coating layer formation stage (b ) or (b₂) is performed preferably under substantially non-aqueous condition.

By electroplating a metal layer on the electrode surface, a large number of micro-pores are formed on the electrode surface; and the enzyme and the mediator for electron transfer are entrapped therein and covered and immobilized with the electropolymerizing substance.

In case wherein the electroplated layer and the electropolymerized layer both containing the enzyme and the mediator for electron transfer, it is possible to effectively perform the electron transfer reaction by the enzyme and so to obtain excellent linearity of sensor output current against enzyme concentration.

On the other hand, in case wherein the electroplated metal layer containing the enzyme and the electropolymerized layer containing the mediator of electron transfer are formed in this order, it is possible to obtain a long lifetime biosensor, since the enzyme is effectively protected by the electropolymerized layer.

### BRIEF DESCRIPTION OF THE DRAWINGS

Fig. 1 shows a schematic illustration of the process steps of the present invention including two courses of (1) Figs. 1A, 1B and 1C and (2) Figs. 1A, 1B, 1D and 1E.

Fig. 2 shows a calibration line of glucose sensor obtained by the process steps of the Figs. 1A, 1B and 1C.

### DESCRIPTION OF THE PREFERRED EMBODIMENTS

The present invention will be explained below further in detail with respect to some of its embodiments shown as working examples.

### Example 1

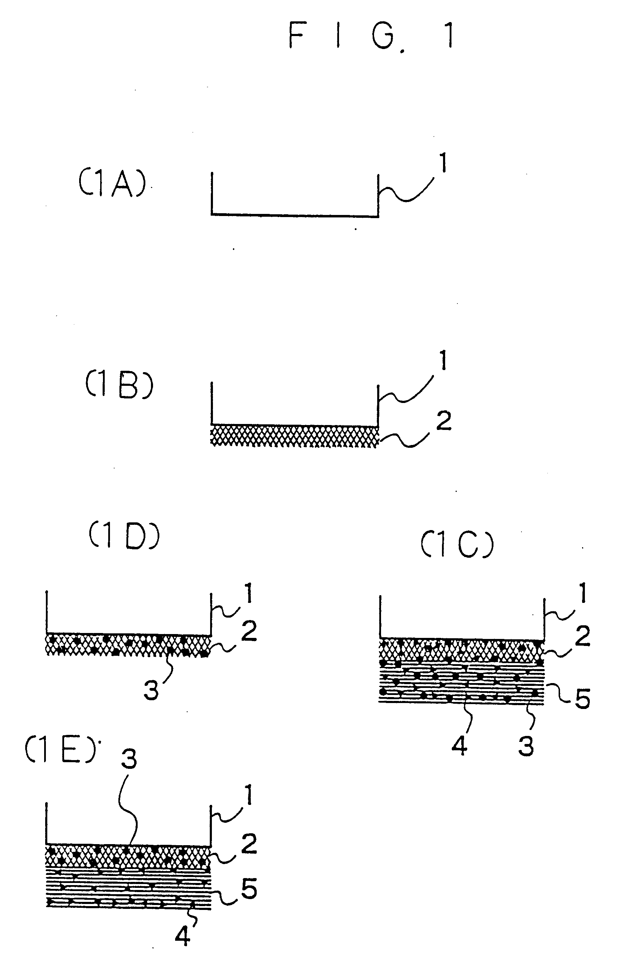

A course of the process steps shown in Figs. 1A, 1B and 1C was taken.

An electrode 1 shown in Fig. 1A was prepared by sputtering titanium and gold on a sapphire substrate of 2.3 x 0.4 mm.

The electrode 1 was immersed in 0.1 M sulfuric acid solution and subjected to a potential sweeping treatment at a sweeping rate of 200 mV/sec for 10 minutes, at an electrode potential of -1.5 to +1.5 V against a saturated calomel electrode as a reference electrode.

The electrode 1 was then electroplated to form a platinum layer 2, as shown in Fig. 1B, by reduction in an aqueous solution of 33 mg/mL of chloroplatinic acid and 0.6 mg/mL of lead acetate at 100 µA for one minute and at 200-225 µA for ten minutes, using a platinum electrode as a counter electrode.

The platinized electrode 1 then was immersed in an acetonitrile solution of 20 mg/mL of glucose oxidase 3 shown in Fig. 1C, 10 mM of ferrocene carboxylic acid 4 shown in Fig. 1C, 0.1 M of tetraethylammonium-p-toluene sulfonic acid, 0.5 M of tetraethylammonium chloride and 0.2 M of pyrrole 5 shown in Fig. 1C and the pyrrole 5 was electropolymerized galvanostatically at electric current density of 3 mA/cm² to form a polymer coating layer thereby to immobilize the glucose oxidase 3 and the ferrocene carboxylic acid 4 on the platinized surface of the electrode 1.

### Example 2

A course of the process steps shown in Figs. 1A, 1B, 1D and 1E was taken.

The procedures up to electroplating of electrode corresponding to Figs. 1A and 1B were the same as those of Example 1.

Then the platinized electrode 1 was immersed in a phosphate buffered solution of pH 7.0 containing 20 mg/mL glucose oxidase 3 shown in Fig. 1D at 4°C for 12 hours to take up the glucose oxidase 3 into the electroplated platinum layer 2. It is possible to control the amount of the glucose oxidase 3 to be taken up into the electroplated platinum layer 2 by charging an electric potential to the electrode 1.

Next, the electrode 1 was immersed in an acetonitrile solution of 10 mM of ferrocene carboxylic acid 4 shown in Fig. 1D, 0.1 M of tetraethylammonium-p-toluene sulfonic acid, 0.5 M of tetraethylammonium chloride and 0.2 M of pyrole 5 shown in Fig. 1D and the pyrrole 5 was electropolymerized galvanostatically at electric current density of 3 mA/cm² to form a polymer coating layer thereby to immobilize the glucose oxidase 3 and the ferrocene carboxylic acid 4 on the platinized surface of the electrode 1.

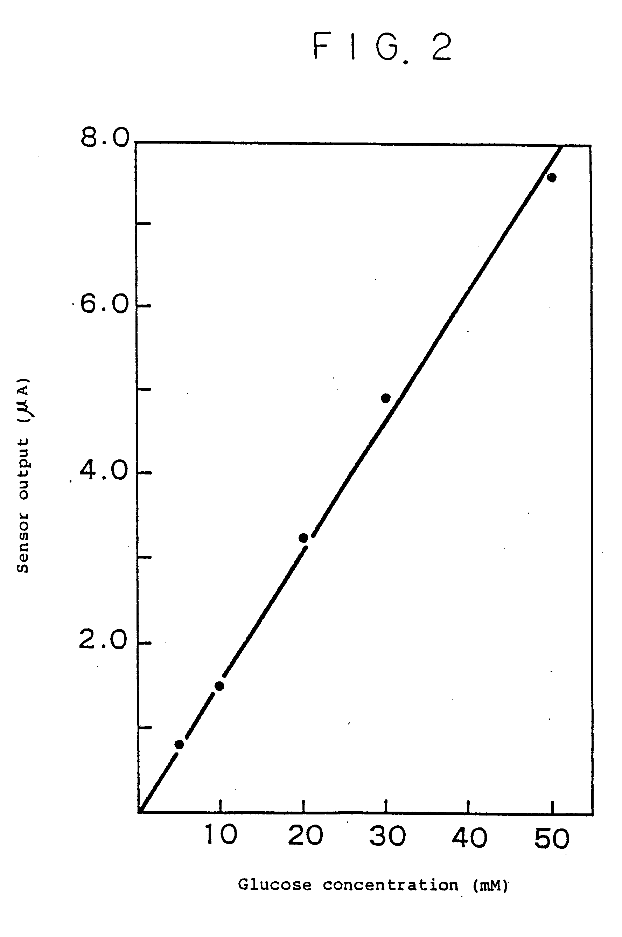

In Fig. 2, a calibration line of the glucose sensor produced in Example 1 is shown. It is seen that straight linearity extends up to 50 mM glucose concentration. This means that the range of linearity has been broadened 2.5 to 5 times of prior art glucose sensors.

In the above Examples, glucose oxidase 3 was used as the enzyme, ferrocene carboxylic acid 4 as the mediator for electron transfer and pyrrole 5 as the electropolymerizing substance. It is also possible however to use any oxidase series enzymes containing a flavin adenine dinucleotide (FAD), such as D-amino-acid oxidase, pyruvate oxidase, choline oxidase, taking advantage of the fact that these enzymes can easily be used for electron transfer reactions owing to FAD. It is also possible to replace the ferrocene carboxylic acid by any other ferrocene derivatives such as 1,1'-dimethyl ferrocene, methyl ferrocene, hydroxymethyl ferrocene. The reason for using ferrocene derivatives is that they facilitate the electron transfer from the enzyme. Further, the pyrrole may be replaced by any other electropolymerizable substances such as N-methylpyrrole, aniline.

As for the solvent for electropolymerization, acetonitrile was used in the above Examples. Other organic solvents, such as nitromethane, N,N-dimethylformamide, N,N-dimethylacetoamide, N,N-diethylacetoamide, N-methylformamide, tetrahydrofuran, formamide or dimethylsulfoxide may also be used, since these are good solvents also for the mediator for electron transfer. Water may also be used, preferably together with a small amount of a surface active agent, but many mediators for electron transfer are difficultly soluble in water.

As explained in detail as above, the present invention has enabled to immobilize sufficient amounts of an enzyme and a mediator of electron transfer, by electroplating the electrode surface to form many micro-pores and by covering the outermost layer of the electrode with an electropolymerized substance. Accordingly, the response range of the sensor has been broadened 2.5 to 5 times and the lifetime of the sensor has been prolonged.

## Claims

1. A process for immobilizing an enzyme on to an electrode surface comprising the steps of:
(a) electroplating a metal layer on the electrode surface, and
(b) forming on the electroplated electrode surface a polymer coating layer by electropolymerization of a solution essentially consisting of the enzyme, a mediator of electron transfer and an electropolymerizing substance.

2. The process for immobilizing an enzyme on an electrode surface comprising the stages of:
(a) electroplating a metal layer on the electrode surface,
(b₁) physically adsorbing the enzyme on to the electroplated electrode surface, and
(b₂) then, forming thereon a polymer coating layer essentially consisting of the mediator of electron transfer and the electropolymerizing substance, by electropolymerization.

3. The process according to claim 1 and 2, wherein the electrode surface is subjected to an electrochemical pretreatment before the electroplating step (a).

4. The process according to claim 2, wherein the physical adsorption stage (b₁) is performed by charging electric potential.

5. The process according to claim 1 or 2, wherein the polymer coating layer formation stage (b ) or (b₂) is performed under substantially non-ageous condition.

6. A biosensor comprising an immobilized enzyme electrode which has, on the electrode surface, a layer of an electroplated metal containing the enzyme and a mediator of electron transfer, and a layer of an electropolymerized substance containing the enzyme and the mediator of electron transfer, in this order.

7. A biosensor comprising an immobilized enzyme electrode which has, on the electrode surface, a layer of an electroplated metal containing the enzyme, and a layer of an electropolymerized substance containing a mediator of electron transfer, in this order.

## Patentansprüche

1. Verfahren zum Immobilisieren eines Enzyms auf einer Elektrodenfläche, welches die folgenden Schritte umfaßt:
(a) Galvanisieren einer Metallschicht auf der Elektrodenfläche, und
(b) Bildung einer Polymerbeschichtung auf der galvanisierten Elektrodenfläche durch Elektropolymerisierung einer im wesentlichen aus dem Enzym, einem Vermittler für die Elektronenübertragung und einer Substanz zur Elektropolymerisation bestehenden Lösung.

2. Verfahren zum Immobilisieren eines Enzyms auf einer Elektrodenfläche, welches die folgenden Phasen umfaßt:
(a) Galvanisieren einer Metallschicht auf der Elektrodenfläche,
(b₁) physikalisches Adsorbieren des Enzyms auf der galvanisierten Elektrodenfläche, und
(b₂) anschließende Bildung einer Polymerbeschichtung darauf, die im wesentlichen aus dem Vermittler für die Elektronenübertragung und der Substanz zur Elektropolymerisation besteht, durch Elektropolymerisierung.

3. Verfahren nach Anspruch 1 und 2, bei welchem die Elektrodenfläche vor dem Galvanisierungsschritt (a) elektrochemisch vorbehandelt wird.

4. Verfahren nach Anspruch 2, bei welchem die Phase der physikalischen Adsorption (b₁) durch Ladung eines elektrischen Potentials durchgeführt wird.

5. Verfahren nach Anspruch 1 oder 2, bei welchem die Phase der Bildung einer Polymerbeschichtung (b bzw. b₂) unter einer im wesentlichen nichtwäßrigen Bedingung durchlaufen wird.

6. Biosensor, der eine Elektrode mit darauf immobilisiertem Enzym umfaßt und in der angegebenen Reihenfolge auf der Elektrodenfläche eine aufgalvanisierte Metallschicht aufweist, die das Enzym und einen Vermittler für die Elektronenübertragung enthält, sowie eine Schicht einer elektropolymerisierten Substanz, welche das Enzym und den Vermittler für die Elektronenübertragung enthält.

7. Biosensor, der eine Elektrode mit darauf immobilisiertem Enzym umfaßt und in der angegebenen Reihenfolge auf der Elektrodenfläche eine aufgalvanisierte Metallschicht aufweist, welche das Enzym enthält, und eine Schicht aus einer elektropolymerisierten Substanz, welche einen Vermittler für die Elektronenübertragung enthält.

## Revendications

1. Procédé pour immobiliser une enzyme sur une surface d'électrode comprenant les étapes consistant à :
(a) revêtir par galvanoplastie une surface de l'électrode avec une couche d'un métal, et
(b) former sur la surface de l'électrode revêtue par galvanoplastie une couche de revêtement en polymère par électropolymérisation d'une solution constituée essentiellement de l'enzyme, d'un médiateur de transfert d'électrons et d'une substance d'électropolymérisation.

2. Procédé pour immobiliser une enzyme sur une surface d'électrode comprenant les stades consistant à :
(a) revêtir par galvanoplastie une surface de l'électrode avec une couche d'un métal,
(b₁) adsorber physiquement l'enzyme sur la surface de l'électrode revêtue par galvanoplastie, et
(b₂) ensuite, former sur son dessus une couche de revêtement en polymère constituée essentiellement du médiateur de transfert d'électrons et de la substance d'électropolymérisation, par électropolymérisation.

3. Procédé selon la revendication 1 ou 2, dans lequel la surface de l'électrode est soumise à un traitement électrochimique préalable avant l'étape de revêtement par galvanoplastie (a).

4. Procédé selon la revendication 2, dans lequel le stade d'adsorption physique (b₁) est exécuté par un potentiel électrique de charge.

5. Procédé selon la revendication 1 ou 2, dans lequel le stade de formation d'une couche de revêtement en polymère (b) ou (b₂) est exécuté dans des conditions pratiquement non aqueuses.

6. Biocapteur comprenant une électrode à enzyme immobilisée qui comporte, sur la surface de l'électrode, une couche d'un métal revêtu par galvanoplastie contenant l'enzyme et d'un médiateur de transfert d'électrons, et une couche d'une substance électropolymérisée contenant l'enzyme et le médiateur de transfert d'électrons, dans cet ordre.

7. Biocapteur comprenant une électrode à enzyme immobilisée qui a, sur la surface de l'électrode, une couche d'un métal revêtu par galvanoplastie contenant l'enzyme, et une couche d'une substance électropolymérisée contenant un médiateur de transfert d'électrons, dans cet ordre.
